# EUROPÄISCHE PATENTANMELDUNG

(11) **EP 0 618 225 A1**
(43) Veröffentlichungstag der Anmeldung: **05.10.1994**
(21) Anmeldenummer: 94104395.2
(22) Anmeldetag: 21.03.1994
(51) Int. Cl.: C07K 5/06, C07K 5/08, C07K 5/10, C07K 7/06, C07K 7/08, A61K 37/02

(54) **Lineare Adhäsionsinhibitoren**

(30) Priorität: 01.04.1993 DE 4310632
(71) Anmelder: MERCK PATENT GmbH, D-64293 Darmstadt (DE)
(72) Erfinder: Jonczyk, Alfred, Dr., D-64295 Darmstadt (DE); Diefenbach, Beate, D-64289 Darmstadt (DE); Felding-Habermann, Brunhilde, Dr., c/o BFH Program, N Torrey Pines Rd, La Jolla, CA 92 037 (US)

(57) **Zusammenfassung**

Die Erfindung betrifft neue lineare Peptide der Formel I

X-A-B-C-Arg-E-G-L-Z I,

worin A, B, C, E, G, L, X und Z die in Anspruch 1 angegebene Bedeutung besitzen, sowie deren Salze.

Diese Verbindungen wirken als Integrin-Inhibitoren und können insbesondere zur Prophylaxe und Behandlung von Erkrankungen des Kreislaufs und der Tumortherapie verwendet werden.

## Beschreibung

Die Erfindung betrifft neue lineare Peptide der Formel I

X-A-B-C-Arg-E-G-L-Z I,

worin
- X: H, Acyl mit 1-10 C-Atomen, H-Asn, H-Val-Asn, H-Asp-Val-Asn, Fmoc-Gly-Gly, H-Lys-Gly-Gly, H-Lys-Pro, H-Tyr-Gly-Gly, H-Cys-Gly-Gly, H-Cys(Trt)-Gly-Gly, H-Cys-Gly-Gly-Thr-Asp-Val-Asn oder H-Thr-Asp-Val-Asn,
- A, B und C: jeweils unabhängig voneinander fehlen oder einen Aminosäurerest, ausgewählt aus einer Gruppe bestehend aus Ala, Arg, Asn, Asp, Asp(OR), Cys, Gln, Glu, Gly, His, Ile, Leu, Lys, Lys(Ac), Lys(AcNH₂), Lys(AcSH), Met, Orn, Phe, 4-Hal-Phe, Pro, Ser, Thr, Trp, Tyr oder Val, wobei die genannten Aminosäurereste auch derivatisiert sein können,
- E: Gly, His oder Leu-His,
- G: fehlt oder Asp oder Asn,
- L: fehlt oder Gly, Ile, Leu oder Leu-Leu,
- Z: NH₂ oder OH,
- Hal: F, Cl, Br, I und
- Ac: Alkanoyl mit 1-10 C-Atomen
bedeuten,
sowie deren physiologisch unbedenkliche Salze.

Ähnliche Verbindungen sind beispielsweise aus der europäischen Patentanmeldung EP 0 406 428 bekannt.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze sehr wertvolle Eigenschaften besitzen. Vor allem wirken sie als Integrin-Inhibitoren, wobei sie insbesondere die Wechselwirkungen der β₃-Integrin-Rezeptoren mit Liganden hemmen. Diese Wirkung kann z.B. nach der Methode nachgewiesen werden, die von J.W. Smith et al. in J. Biol. Chem. 265, 12267-12271 (1990) beschrieben wird. Zusätzlich treten antiinflammatorische Effekte auf. Auch diese Wirkung kann mit Hilfe von literaturbekannten Methoden nachgewiesen werden.

Die Verbindungen können als Arzneimittelwirkstoffe in der Human- und Veterinärmedizin eingesetzt werden, insbesondere zur Prophylaxe und zur Behandlung von Erkrankungen des Kreislaufs, bei Thrombose, Herzinfarkt, Arteriosklerose, Entzündungen, Apoplexie, Angina pectoris, Tumoren, osteolytischen Erkrankungen, insbesondere Osteoporose, Angiogenese und Restenose nach Angioplastie. Ferner können sie unterstützend bei Wundheilungsprozessen wirken.

Die Verbindungen eignen sich zudem als antimikrobielle Wirkstoffe der Infektionen, wie sie beispielsweise durch Bakterien, Pilze oder Hefen ausgelöst werden können, verhindern. Die Substanzen können daher vorzugsweise als begleitende antimikrobielle Wirkstoffe gegeben werden, wenn Eingriffe an Organismen vorgenommen werden, bei denen körperfremde Stoffe, wie z.B. Biomaterialien, Implantate, Katheter oder Herzschrittmacher, eingesetzt werden. Sie wirken als Antiseptika.

Die vor- und nachstehend aufgeführten Abkürzungen von Aminosäureresten stehen für die Reste folgender Aminosäuren:
- Ala: Alanin
- Arg: Arginin
- Asn: Asparagin
- Asp: Asparaginsäure
- Asp(OR): Asparaginsäure(β-ester)
- Cit: Citrullin
- Cys: Cystein
- Dab: 2,4-Diaminobuttersäure
- Gln: Glutamin
- Glu: Glutaminsäure
- Gly: Glycin
- His: Histidin
- Ile: Isoleucin
- Leu: Leucin
- Lys: Lysin
- Lys(Ac): Nε-Acyllysin
- Lys(AcNH₂): Nε-Aminoacyllysin
- Lys(AcSH): Nε-Mercaptoacyllysin
- Met: Methionin
- Orn: Ornithin
- Phe: Phenylalanin
- 4-Hal-Phe: 4-Halogenphenylalanin
- Pro: Prolin
- Ser: Serin
- Thr: Threonin
- Trp: Tryptophan
- Tyr: Tyrosin
- Val: Valin.

Ferner bedeuten nachstehend:
- BOC: tert-Butoxycarbonyl
- CBZ: Benzyloxycarbonyl
- DCCI: Dicyclohexylcarbodiimid
- DMF: Dimethylformamid
- EDCI: N-Ethyl-N'-(3-dimethylaminopropyl)-carbodiimidhydrochlorid
- Et: Ethyl
- Fmoc: 9-Fluorenylmethoxycarbonyl
- HOBt: 1-Hydroxybenzotriazol
- Me: Methyl
- MBHA: 4-Methyl-benzhydrylamin
- Mtr: 4-Methoxy-2,3,6-trimethylphenyl-sulfonyl
- OBut: tert.-Butylester
- OMe: Methylester
- OEt: Ethylester
- POA: Phenoxyacetyl
- TFA: Trifluoressigsäure.
- Trt: Trityl (Triphenylmethyl)
Sofern die vorstehend genannten Aminosäuren in mehreren enantiomeren Formen auftreten können, so sind vor- und nachstehend, z.B. als Bestandteil der Verbindungen der Formel I, alle diese Formen und auch ihre Gemische (z.B. die DL-Formen) eingeschlossen. Ferner können die Aminosäuren bzw. die Aminosäurereste in an sich bekannter Form derivatisiert sein.

Gegenstand der Erfindung ist ferner ein Verfahren zur Herstellung einer Verbindung der Formel I nach Anspruch 1 oder eines ihrer Salze, dadurch gekennzeichnet, daß man sie aus einem ihrer funktionellen Derivate durch Behandeln mit einem solvolysierenden oder hydrogenolysierenden Mittel in Freiheit setzt
oder daß man ein Peptid der Formel II

M-OH II

worin
- M: (a) X, aber nicht Wasserstoff, Fmoc-Gly, H-Lys-Gly, H-Lys, H-Tyr-Gly, H-Asp-Val, H-Tyr, H-Val, H-Asp, H-Cys-Gly-Gly-Thr-Asp-Val, H-Cys-Gly-Gly-Thr-Asp, H-Cys-Gly-Gly-Thr, H-Cys-Gly-Gly, H-Cys-Gly, H-Cys, H-Cys(Trt)-Gly, H-Cys(Trt), H-Thr-Asp-Val, H-Thr-Asp oder H-Thr,
(b) X-A,
(c) X-A-B,
(d) X-A-B-C,
(e) X-A-B-C-Arg,
(f) X-A-B-C-Arg-E oder
(g) X-A-B-C-Arg-E-G
bedeutet,
mit einer Aminoverbindung der Formel III

H-Q-Z III

worin Z die angegebene Bedeutung besitzt und gegebenenfalls durch eine entsprechende Schutzgruppe geschützt sein kann und
- Q: (a) A-B-C-Arg-E-G-L, Asn-A-B-C-Arg-E-G-L Val-Asn-A-B-C-Arg-E-G-L, Pro-A-B-C-Arg-E-G-L, Gly-A-B-C-Arg-E-G-L, Gly-Gly-A-B-C-Arg-E-G-L, Asn-A-B-C-Arg-E-G-L, Val-Asn-A-B-C-Arg-E-G-L Asp-Val-Asn-A-B-C-Arg-E-G-L, Thr-Asp-Val-Asn-A-B-C-Arg-E-G-L, Gly-Thr-Asp-Val-Asn-A-B-C-Arg-E-G-L oder Gly-Gly-Thr-Asp-Val-Asn-A-B-C-Arg-E-G-L,
(b) B-C-Arg-E-G-L,
(c) C-Arg-E-G-L,
(d) Arg-E-G-L,
(e) E-G-L,
(f) G-L oder
(g) L
bedeutet,
umsetzt
und/oder daß man gegebenenfalls eine freie Aminogruppe acyliert und/oder eine Verbindung der Formel I durch Behandeln mit einer Saure oder einer Base in eines ihrer Salze überführt.

Vor- und nachstehend haben die Reste A, B, C, E, G, L, M, Q, X und Z die bei den Formeln I, II und III angegebenen Bedeutungen, sofern nicht ausdrücklich etwas anderes angegeben ist.

In den vorstehenden Formeln steht Alkyl vorzugsweise für Methyl, Ethyl, Isopropyl oder tert.-Butyl.

X ist vorzugsweise Wasserstoff, H-Asn, Fmoc-Gly-Gly oder H-Cys(Trt)-Gly-Gly, besonders bevorzugt aber H-Cys-Gly-Gly. Ferner kann X auch Acyl mit 1-10 C-Atomen sein, wobei Acyl vorzugsweise Alkanoyl mit 1-8, insbesondere 1, 2, 3, oder 4 C-Atomen bedeutet, in einzelnen bevorzugt Formyl, Acetyl, Propionyl, Butyryl, Isobutyryl, Pentanoyl oder Hexanoyl, aber auch Aryl-CO, wie z.B. Benzoyl, o-, m- oder p-Toluyl, o-, m- oder p-Methoxybenzoyl sowie 2-Naphthoyl oder aber Aryl-CₙH₂ₙ-CO (n = 1-4), wie z.B. Ph-CH₂-CO, Ph(CH₂)₂-CO, Ph-(CH₂)₃CO oder Ph-(CH₂)₄-CO (Ph = Phenyl), wobei Ph auch durch eine OCH₃- oder CH₃-Gruppe substituiert sein kann.

Die Gruppe A ist vorzugsweise Ala, Leu, Ser, insbesondere Gly oder aber nicht vorhanden. B ist vorzugsweise Asn oder Asp. C ist vorzugsweise Ala, auch D-Ala, His und insbesondere Gly. E ist vorzugsweise Gly oder His. G ist bevorzugt Asp, während L vorzugsweise für Leu steht. Z bedeutet NH₂, besonders bevorzugt aber OH.

Dementsprechend sind Gegenstand der Erfindung insbesondere diejenigen Verbindungen der Formel I, in denen mindestens einer der genannten Reste eine der vorstehend angegebenen bevorzugten Bedeutungen hat.

Eine bevorzugte Gruppe von Verbindungen kann durch die Formel Ia ausgedrückt werden, die der Formel I entspricht und worin B, C, E, X und Z die dort angegebenen Bedeutungen besitzen und
- A: Gly,
- G: Asp und
- L: Leu bedeuten.

Eine weitere Gruppe von bevorzugten Verbindungen kann durch die Teilformeln Iaa bis Iad ausgedrückt werden, die sonst der Formel I bzw. Ia entsprechen, worin jedoch in Iaa:
- X: Wasserstoff oder Acetyl
- A: Gly,
- B: Asn oder Asp,
- C: fehlt, Ala oder Gly,
- E: Gly oder His,
- G: Asp,
- L: Leu und
- Z: NH₂ oder OH,
in Iab:
- X: Wasserstoff oder Acyl,
- A: fehlt oder Gly,
- B: fehlt oder Asn,
- C: Gly,
- E: Gly oder His,
- G: Asp,
- L: Leu und
- Z: NH₂ oder OH,
in Iac:
- X: Wasserstoff,
- A: fehlt oder Gly
- B: fehlt oder Asn
- C: fehlt oder Gly
- E: Gly oder His
- G: Asp
- L: Leu und
- Z: OH oder NH₂,
in Iad:
- X: H-Asn, H-Val-Asn, H-Asp-Val-Asn, Fmoc-Gly-Gly, H-Lys-Gly-Gly, H-Tyr-Gly-Gly, H-Cys(Trt)-Gly-Gly, H-Cys-Gly-Gly, H-Cys-Gly-Gly-Thr-Asp-Val-Asn oder H-Thr-Asp-Val-Asn,
- A: Gly,
- B: Asn,
- C: Gly,
- E: Gly oder His,
- G: Asp,
- L: Leu und
- Z: NH₂ oder OH
bedeuten.

Besonders geeignete Verbindungen sind solche, die der Formel I entsprechen und worin A, B, C, X und Z die zuvor angegebenen bevorzugten Bedeutungen besitzen, worin aber der zentrale Aminosäurerest für L-Arg, nicht für D-Arg, E für Gly sowie His, nicht für D-His, G für Asp und L für Leu stehen.

Die Verbindungen der Formel I und auch die Ausgangsstoffe zu ihrer Herstellung werden im übrigen nach bekannten Methoden hergestellt, wie sie in der Literatur (z. B. in den Standardwerken wie Houben-Weyl, Methoden der organischen Chemie, Georg-Thieme-Verlag, Stuttgart) beschrieben sind und zwar unter Reaktionsbedingungen, die für die genannten Umsetzungen bekannt und geeignet sind. Dabei kann man auch von bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Die Verbindungen der Formel I können erhalten werden, indem man sie aus ihren funktionellen Derivaten durch Solvolyse, insbesondere Hydrolyse, oder durch Hydrogenolyse in Freiheit setzt.

Bevorzugte Ausgangsstoffe für die Solvolyse bzw. Hydrogenolyse sind solche, die anstelle einer oder mehrerer freier Amino- und/oder Hydroxygruppen entsprechende geschützte Amino- und/oder Hydroxygruppen enthalten, vorzugsweise solche, die anstelle eines H-Atoms, das mit einem N-Atom verbunden ist, eine Aminoschutzgruppe tragen, z. B. solche, die der Formel I entsprechen, aber anstelle einer NH₂-Gruppe eine NHR'-Gruppe (worin R' eine Aminoschutzgruppe bedeutet, z. B. BOC oder CBZ) enthalten.

Ferner sind Ausgangsstoffe bevorzugt, die anstelle des H-Atoms einer Hydroxygruppe eine Hydroxyschutzgruppe tragen, z. B. solche, die der Formel I entsprechen, aber anstelle einer Hydroxyphenylgruppe eine R''O-phenylgruppe enthalten (worin R'' eine Hydroxyschutzgruppe bedeutet).

Es können auch mehrere - gleiche oder verschiedene - geschützte Amino- und/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an anderen Stellen des Moleküls duchgeführt worden ist. Typisch für solche Gruppen sind insbesondere unsubstituierte oder substituierte Acyl-, Aryl-, Aralkoxymethyl- oder Aralkylgruppen. Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren und den vorliegenden Verbindungen in weitestem Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie POA; Alkoxycarbonyl wie Methoxycarbonyl, Ethoxycarbonyl, 2,2,2- Trichlorethoxycarbonyl, BOC, 2-Jodethoxycarbonyl; Aralkyloxycarbonyl wie CBZ ("Carbobenzoxy"), 4-Methoxybenzyloxycarbo- nyl, FMOC; Arylsulfonyl wie Mtr. Bevorzugte Aminoschutzgruppen sind BOC und Mtr, ferner CBZ, Fmoc, Benzyl und Acetyl.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an anderen Stellen des Moleküls durchgeführt worden ist. Typisch für solche Gruppen sind die oben genannten unsubstituierten oder substituierten Aryl-, Aralkyl- oder Acylgruppen, ferner auch Alkylgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. Benzyl, p-Nitrobenzoyl, p-Toluolsulfonyl und Acetyl, wobei Benzyl und Acetyl besonders bevorzugt sind. Die COOH-Gruppen ins Asparaginsäure und Glutaminsäure werden bevorzugt in Form ihrer tert.-Butylester geschützt (z.B. Asp (OBut)).

Die als Ausgangsstoffe zu verwendenden funktionellen Derivate der Verbindungen der Formel I können nach üblichen Methoden der Aminosäure- und Peptidsynthese hergestellt werden, wie sie z. B. in den genannten Standardwerken und Patentanmeldungen beschrieben sind, z. B. auch nach der Festphasenmethode nach Merrifield (B.F. Gysin u. R.B. Merrifield, J. Am. Chem. Soc. 94, 3102ff. (1972)).

Das In-Freiheit-Setzen der Verbindungen der Formel I aus ihren funktionellen Derivaten gelingt - je nach der benutzten Schutzgruppe - z. B. mit starken Säuren, zweckmäßig mit TFA oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure Die Anwesenheit eines zusätzlichen inerten Lösungsmittels ist möglich, aber nicht immer erforderlich. Als inerte Lösungsmittel eignen sich vorzugsweise organische, beispielsweise Carbonsäuren wie Essigsäure, Ether wie Tetrahydrofuran oder Dioxan, Amide wie DMF, halogenierte Kohlenwasserstoffe wie Dichlormethan, ferner auch Alkohole wie Methanol, Ethanol oder Isopropanol sowie Wasser. Ferner kommen Gemische der vorgenannten Lösungsmittel in Frage. TFA wird vorzugsweise im Überschuß ohne Zusatz eines weiteren Lösungsmittels verwendet, Perchlorsäure in Form eines Gemisches aus Essigsäure und 70%iger Perchlorsäure im Verhältnis 9 : 1. Die Reaktionstemperaturen für die Spaltung liegen zweckmäßig zwischen etwa 0 und etwa 50°, vorzugsweise arbeitet man zwischen 15 und 30° (Raumtemperatur).

Die Gruppen BOC, OBut und Mtr können z. B. bevorzugt mit TFA in Dichlormethan oder mit etwa 3 bis 5 n HCl in Dioxan bei 15-30° abgespalten werden, die Fmoc-Gruppe mit einer etwa 5- bis 50%igen Lösung von Dimethylamin, Diethylamin oder Piperidin in DMF bei 15-30°.

Hydrogenolytisch entfernbare Schutzgruppen (z. B. CBZ oder Benzyl) können z. B. durch Behandeln mit Wasserstoff in Gegenwart eines Katalysators (z. B. eines Edelmetallkatalysators wie Palladium, zweckmäßig auf einem Träger wie Kohle) abgespalten werden. Als Lösungsmittel eignen sich dabei die oben angegebenen, insbesondere z. B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z. B. gut an 5 bis 10%igem Pd-C in Methanol oder mit Anmoniumformiat (anstelle von H₂) an Pd-C in Methanol/DMF bei 20-30°.

Verbindungen der Formel I können auch durch Umsetzung einer Verbindung der Formel II mit einer Aminoverbindung der Formel III unter für Peptidsynthesen an sich bekannten, kondensierenden Bedingungen, wie sie z.B. in Houben-Weyl, l.c., Bd. 1-5/II, S. 1-806 (1974) beschrieben sind, erhalten werden.

Die Reaktion gelingt vorzugsweise in Gegenwart eines Dehydratisierungsmittels, z. B. eines Carbodiimids wie DCCI oder EDCI, ferner Propanphosphonsäureanhydrid (vgl. Angew.Chem. 92, 129 (1980)), Diphenylphosphorylazid oder 2-Ethoxy-N-ethoxycarbonyl-1,2-dihydrochinolin, in einem inerten Lösungsmittel, z. B. einem halogenierten Kohlenwasserstoff wie Dichlormethan, einem Ether wie Tetrahydrofuran oder Dioxan, einem Amid wie DMF oder Dimethylacetamid, einem Nitril wie Acetonitril, oder in Gemischen dieser Lösungsmittel, bei Temperaturen zwischen etwa -10 und 40, vorzugsweise zwischen 0 und 30°.

Anstelle von II können auch geeignete reaktionsfähige Derivate dieser Stoffe in die Reaktion eingesetzt werden, z.B. solche, in denen reaktive Gruppen intermediär durch Schutzgruppen blockiert sind. Die Aminosäurederivate II können z.B. in Form ihrer aktivierten Ester verwendet werden, die zweckmäßig in situ gebildet werden, z. B. durch Zusatz von HOBt oder N-Hydroxysuccinimid.

Die Ausgangsstoffe der Formel II sind in der Regel neu. Sie können nach bekannten Methoden, z. B. den oben angegebenen Methoden der Peptidsynthese und der Abspaltung von Schutzgruppen, hergestellt werden.

In der Regel synthetisiert man zunächst geschützte Peptidester der Formel R'-M'-OR'', wobei M' dem am N-terminalen Ende um ein H-Atom reduzierten Rest M entspricht, z.B. BOC-M'-OMe oder Fmoc-M'-OMe. Diese werden zu Säuren der Formel R'-M'-OH, z.B. BOC-M'-OH oder Fmoc-M'-OH verseift und dann mit einer Verbindung der Formel III, die gegebenenfalls ebenfalls mit entsprechende Schutzgruppen an Positionen, die der Reaktion nicht zugänglich sein sollen, versehen ist, kondensiert.

Im Falle von Verbindungen der Formel III synthetisiert man ebenfalls Peptidester der Formel R'-Q-Z'-R'', wie z.B. BOC-Q-Z'-OMe oder Fmoc-Q-Z'-OMe, wobei Z'-NH- oder -O- bedeutet und spaltet dann, ehe man die Kondensation zur Herstellung von Verbindungen der Formel I durchführt, die Schutzgruppe R' auf bekannte Weise, z.B. Fmoc durch Behandlung mit einer Piperidin/DMF-Lösung, ab.

Besonders vorteilhaft können die neueren Methoden der Peptidsynthese nach modifizierten Merrifield-Techniken und unter Verwendung von Peptidsynthesegeräten, wie sie z.B. in Peptides, Proc. 8th Am. Pept. Symp., Eds. V. Hruby und D.H. Rich, Pierce Comp. III, p. 73-77 (1983) von A. Jonczyk und J. Meienhofer (Fmoc-Strategie) beschrieben sind oder die in Angew. Chem. 104, 375-391 (1992) dargestellten Techniken, Verwendung finden. Derartige Methoden sind an sich bekannt und deren Schilderung daher an dieser Stelle entbehrlich.

Eine Base der Formel I kann mit einer Säure in das zugehörige Säureadditionssalz übergeführt werden. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2- oder 3-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono-und -disulfonsäuren, Laurylschwefelsäure. Salze mit physiologisch nicht unbedenklichen Säuren, z.B. Pikrate, können zur Isolierung und/oder Aufreinigung der Verbindungen der Formel I verwendet werden.

Andererseits kann eine Säure der Formel I durch Umsetzung mit einer Base in eines ihrer physiologisch unbedenklichen Metall- oder Ammoniumsalze übergeführt werden. Als Salze kommen dabei insbesondere die Natrium-, Kalium-, Magnesium-, Calcium- und Ammoniumsalze in Betracht, ferner substituierte Ammoniumsalze, z.B. die Dimethyl-, Diethyl- oder Diisopropylammoniumsalze, Monoethanol-, Diethanol- oder Triethanolammoniumsalze, Cyclohexyl-, Dicyclohexylammoniumsalze, Dibenzylethylendiammoniumsalze, weiterhin z.B. Salze mit N-Methyl-D-glucamin oder mit Arginin oder Lysin.

Ferner können die neuen Verbindungen der Formel I als Integrinliganden zur Herstellung von Säulen für die Affinitätschromatographie zur Reindarstellung von Integrinen verwendet werden.

Der Ligand, d.h. ein Peptidderivat der Formel I, wird dabei über Ankerfunktionen an einen polymeren Träger kovalent gekuppelt.

Als polymere Trägermaterialien eignen sich die an sich in der Peptidchemie bekannten polymeren festen Phasen mit vorzugsweise hydrophilen Eigenschaften, beispielsweise quervernetzte Polyzucker, wie Zellulose, Sepharose oder Sephadex®, Acrylamide, Polymere auf Polyethylenglykolbasis oder Tentakelpolymere®.

Als Ankerfunktionen, die mit den polymerern Trägern verknüpft sind, eignen sich vorzugsweise lineare Alkylenketten mit 2-12 C-Atomen, die mit einem Ende direkt an das Polymer gebunden sind und am anderen Ende eine funktionelle Gruppe, wie z.B. Hydroxy, Amino, Mercapto, Maleinimido oder -COOH aufweisen und dazu geeignet sind, mit dem C- oder N-terminalen Abschnitt des jeweiligen Peptids verknüpft zu werden.

Dabei ist es möglich, daß das Peptid direkt oder ebenfalls über eine zweite Ankerfunktion mit dem Anker des Polymers verbunden ist. Ferner ist es möglich, daß Peptide, die Aminosäurereste mit funktionalisierten Seitenketten enthalten, über diese mit der Ankerfunktion des Polymers verbunden werden.

Darüber hinaus können bestimmte Aminosäurereste, die Bestandteil der Peptide der Formel I sind, in ihren Seitenketten derart modifiziert werden, daß sie zur Verankerung über z.B. SH-, OH-, NH₂- oder COOH-Gruppen mit dem Anker des Polymers zur Verfügung stehen.

Möglich sind hierbei ungewöhnliche Aminosäuren, wie z.B. Phenylalaninderivate, die in 4-Position des Phenylrings eine Mercapto-, Hydroxy-, Amino- oder Carboxyalkylkette tragen, wobei die funktionelle Gruppe sich am Ende der Kette befindet..

Beispiele für Aminosäurereste, deren Seitenkette direkt als Ankerfunktion dienen kann, sind z.B. Lys, Orn, Arg, Dab, Asp, Asn, Glu, Gln, Ser, Thr, Cys, Cit oder Tyr.

Beispiele für N-terminale Anker sind Reste, wie z.B. -CO-CₙH₂ₙ-NH₂, -CO-CₙH₂ₙ-OH, -CO-CₙH₂ₙ-SH oder -CO-CₙH₂ₙ-COOH mit n = 2-12, wobei die Länge der Alkylenkette nicht kritisch ist und diese gegebenenfalls auch z.B. durch entsprechende Aryl- oder Alkylarylreste teilweise oder vollständig ersetzt werden kann.

C-terminale Anker können beispielsweise -O-CₙH₂ₙ-SH, -O-CₙH₂ₙ-OH, -O-CₙH₂ₙ-NH₂, -O-CₙH₂ₙ-COOH, -NH-CₙH₂ₙ-SH, -NH-CₙH₂ₙ-OH, -NH-CₙH₂ₙ-NH₂ oder -NH-CₙH₂ₙ-COOH sein, wobei für n sowie für die Alkylenkette das bereits im vorhergehenden Abschnitt gesagte gilt.

Die N- und C-terminalen Anker können auch als Ankerbaustein für eine bereits funktionalisierte Seitenkette eines Aminosäurerests dienen. Es kommen hier beispielsweise Aminosäurereste wie Lys(CO-C₅H₁₀-NH₂), Asp(NH-C₃H₆-COOH) oder Cys(C₃H₆-NH₂) in Frage, wobei der Anker immer an die funktionelle Gruppe der Seitenkette gebunden ist.

Die Herstellung der Materialien für die Affinitätschromatographie erfolgt unter Bedingungen wie sie für die Kondensation von Aminosäuren üblich und an sich bekannt sind und bereits im Abschnitt zur Herstellung der Verbindungen der Formel I geschildert wurden, bzw. in Pierce, Immuno Technology Catalog & Handbook (1990) beschrieben sind.

Die neuen Verbindungen der Formel I und ihre physiologisch unbedenklichen Salze können zur Herstellung pharmazeutischer Präparate verwendet werden, indem man sie zusammen mit mindestens einem Träger- oder Hilfsstoff und, falls erwünscht, zusammen mit einem oder mehreren weiteren Wirkstoff(en) in eine geeignete Dosierungsform bringt. Die so erhaltenen Zubereitungen können als Arzneimittel in der Human- oder Veterinärmedizin eingesetzt werden. Als Trägersubstanzen kommen organische oder anorganische Stoffe in Frage, die sich für die enterale (z.B. orale oder rektale), parenterale (z.B. intravenöse Injektion) oder lokale (z.B. topische, dermale, ophthalmische oder nasale) Applikation oder für eine Applikation in Form eines Inhalations-Sprays eignen und mit den neuen Verbindungen nicht reagieren, beispielsweise Wasser oder wässerige isotonische Kochsalzlösung, niedere Alkohole, pflanzliche Öle, Benzylalkohole, Polyethylenglykole, Glycerintriacetat und andere Fettsäureglyceride, Gelatine, Sojalecithin, Kohlehydrate wie Lactose oder Stärke, Magnesiumstearat, Talk, Cellulose, Vaseline. Zur oralen Anwendung dienen insbesondere Tabletten, Dragees, Kapseln, Sirupe, Säfte oder Tropfen; von Interesse sind speziell Lacktabletten und Kapseln mit magensaftresistenten Überzügen bzw. Kapselhüllen. Zur rektalen Anwendung dienen Suppositorien, zur parenteralen Applikation Lösungen, vorzugsweise ölige oder wässerige Lösungen, ferner Suspensionen, Emulsionen oder Implantate. Zur topischen Anwendung eignen sich z.B. Lösungen, die in Form von Augentropfen verwendet werden können, ferner z. B. Suspensionen, Emulsionen, Cremes, Salben oder Komprimate. Für die Applikation als Inhalations-Spray können Sprays verwendet werden, die den Wirkstoff entweder gelöst oder suspendiert in einem Treibgas oder Treibgasgemisch (z.B. CO₂ oder Fluorchlorkohlenwasserstoffen bzw. geeignete Ersatzstoffe) enthalten. Zweckmäßig verwendet man den Wirkstoff dabei in mikronisierter Form, wobei ein oder mehrere zusätzliche physiologisch verträgliche Lösungsmittel zugegen sein können, z.B. Ethanol. Inhalationslösungen können mit Hilfe üblicher Inhalatoren verabfolgt werden. Die neuen Verbindungen können auch lyophilisiert und die erhaltenen Lyophilisate z.B. zur Herstellung von Injektionspräparaten verwendet werden. Die Injektionen können dabei als Bolus oder als kontinuierliche Infusion (z.B. intravenös, intramusculär, subcutan oder intrathecal) gegeben werden. Die angegebenen Zubereitungen können sterilisiert sein und/oder Hilfsstoffe wie Konservie rungs-, Stabilisierungs und/oder Netzmittel, Emulgatoren, Salze zur Beeinflussung des osmotischen Druckes, Puffersubstanzen, Farb- und/oder Aromastoffe enthalten. Sie können, falls erwünscht, auch einen oder mehrere weitere Wirkstoffe enthalten, z. B. ein oder mehrere Vitamine.

Die erfindungsgemäßen Substanzen können in der Regel in Analogie zu anderen bekannten, im Handel befindlichen Peptiden, insbesondere aber in Analogie zu den in der US-A-4 472 305 beschriebenen Verbindungen verabreicht werden, vorzugsweise in Dosierungen zwischen etwa 0,05 und 500, insbesondere zwischen 0,5 und 100 mg pro Dosierungseinheit. Die tägliche Dosierung liegt vorzugsweise zwischen etwa 0,01 und 2 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die parenterale Applikation ist bevorzugt.

Vor- und nachstehend sind alle Temperaturen in °C angegeben. In den nachfolgenden Beispielen bedeutet "übliche Aufarbeitung": Man gibt, falls erforderlich, Wasser hinzu, neutralisiert, extrahiert mit Ether oder Dichlormethan, trennt ab, trocknet die organische Phase über Natriumsulfat, filtriert, dampft ein und reinigt durch Chromatographie an Kieselgel und/oder Kristallisation. RZ = Retentionszeit (Minuten) bei HPLC an Lichrosorb RP® select B (250-4,7 µm)-Säule, Laufmittel: 0,3 % TFA in Wasser; Isopropanolgradient von 0-80 Vol% in 50 Min. bei 1 ml/Min. Fluß und Detektion bei 215 nm. M⁺ = Molekular-Peak im Massenspektrum, erhalten nach der "Fast Atom Bombardment"-Methode (FAB), steht in der Regel für M⁺ + H, also die um 1 Masseneinheit erhöhte Masse der jeweiligen Verbindung.

### Beispiel 1

2,2 g BOC-Asp-Gly-OH werden in einer Mischung aus 150 ml Dichlormethan und 20 ml DMF gelöst, auf 0° abgekühlt und anschließend mit 0,5 g DCCI, 0,3 g HOBt, 0,23 ml N-Methylmorpholin und 1 Äquivalent H-Arg-His-Asp-Leu-OMe [beide Peptide sind nach Methoden der modifizierten Merrifield-Techniken erhältlich) versetzt. Es wird 20 Stunden bei 0° und 6 Stunden bei Raumtemperatur gerührt. Man engt die Reaktionsmischung ein, behandelt sie mit einem Mischbett-Ionenaustauscher und gibt sie in eine wäßrige NaHCO₃-Lösung. Das ausfallende Produkt wird abgesaugt und mit Wasser gewaschen. Nach Waschen mit Ethylacetat/Petrolether erhält man BOC-Asp-Gly-Arg-His-Asp-Leu-OMe.

Analog erhält man durch Kondensation von H-Arg-His-Asp-Leu-OMe
mit BOC-Gly-OH:
BOC-Gly-Arg-His-Asp-Leu-OMe;
mit BOC-Asn-Gly-Asp-Gly-OH:
BOC-Asn-Gly-Asp-Gly-Arg-His-Asp-Leu-OMe;
mit BOC-Val-Asn-Gly-Asp-Gly-OH:
BOC-Val-Asn-Gly-Asp-Gly-Arg-His-Asp-Leu-OMe;
mit BOC-Asp-Val-Asn-Gly-Asp-Gly-OH:
BOC-Asp-Val-Asn-Gly-Asp-Gly-Arg-His-Asp-Leu-OMe;
mit BOC-Lys-Gly-Gly-Gly-Asp-Gly-OH:
BOC-Lys-Gly-Gly-Gly-Asp-Gly-Arg-His-Asp-Leu-OMe;
mit BOC-Tyr-Gly-Gly-Gly-Asp-Gly-OH:
BOC-Tyr-Gly-Gly-Gly-Asp-Gly-Arg-His-Asp-Leu-OMe;
mit BOC-Ala-Asp-Gly-OH:
BOC-Ala-Asp-Gly-Arg-His-Asp-Leu-OMe;
mit BOC-D-Ala-Asp-Gly-OH:
BOC-D-Ala-Asp-Gly-Arg-His-Asp-Leu-OMe;
mit BOC-Gly-Asp-Ala-OH:
BOC-Gly-Asp-Ala-Arg-His-Asp-Leu-OMe;
mit BOC-Gly-D-Asp-Gly-OH:
BOC-Gly-D-Asp-Gly-Arg-His-Asp-Leu-OMe;
mit BOC-Gly-Asp-D-Ala-OH:
BOC-Gly-Asp-D-Ala-Arg-His-Asp-Leu-OMe;
mit BOC-Gly-Asp-OH:
BOC-Gly-Asp-Arg-His-Asp-Leu-OMe;
mit BOC-Cys(Trt)-Gly-Gly-Gly-Asp-OH:
BOC-Cys(Trt)-Gly-Gly-Gly-Asp-Arg-His-Asp-Leu-OMe;
mit BOC-Cys-Gly-Gly-Gly-Asp-OH:
BOC-Cys-Gly-Gly-Gly-Asp-Arg-His-Asp-Leu-OMe;
mit BOC-Cys(Trt)-Gly-Gly-Gly-Asp-Gly-OH: 
BOC-Cys(Trt)-Gly-Gly-Gly-Asp-Gly-Arg-His-Asp-Leu-
OMe;
mit BOC-Cys-Gly-Gly-Gly-Asp-Gly-OH:
BOC-Cys-Gly-Gly-Gly-Asp-Gly-Arg-His-Asp-Leu-OMe;
mit BOC-Cys-Gly-Gly-Thr-Asp-Val-Asn-Gly-Asp-Gly-OH: 
BOC-Cys-Gly-Gly-Thr-Asp-Val-Asn-Gly-Asp-Gly-Arg-His-
Asp-Leu-OMe;
mit BOC-Thr-Asp-Val-Asn-Gly-Asp-Gly-OH:

BOC-Thr-Asp-Val-Asn-Gly-Asp-Gly-Arg-His-Asp-Leu-
OMe.

### Beispiel 2

Analog Beispiel 1 erhält man durch Umsetzung von BOC-Gly-Asp-Gly-OH
mit H-Arg-His-Asp-OMe:
BOC-Gly-Asp-Gly-Arg-His-Asp-OMe;
mit H-Arg-His-OMe:
BOC-Gly-Asp-Gly-Arg-His-OMe;
mit H-Arg-His-Asn-Leu-OMe:
BOC-Gly-Asp-Gly-Arg-His-Asn-Leu-OMe;
mit H-Arg-Gly-Asp-Leu-OMe:
BOC-Gly-Asp-Gly-Arg-Gly-Asp-Leu-OMe;
mit H-Arg-His-D-Asp-Leu-OMe:
BOC-Gly-Asp-Gly-Arg-His-D-Asp-Leu-OMe;
mit H-Arg-D-His-Asp-Leu-OMe:
BOC-Gly-Asp-Gly-Arg-D-His-Asp-Leu-OMe;
mit H-D-Arg-His-Asp-Leu-OMe:
BOC-Gly-Asp-Gly-D-Arg-His-Asp-Leu-OMe.

Analog Beispiel 1 erhält man durch Umsetzung von BOC-Gly-Asn-Gly-OH
mit H-Arg-His-Asn-Leu-OMe:
BOC-Gly-Asn-Gly-Arg-His-Asn-Leu-OMe;
mit H-Arg-His-Asp-Leu-OMe:
BOC-Gly-Asn-Gly-Arg-His-Asp-Leu-OMe.

### Beispiel 3

Analog Beispiel 1 erhält man durch Kondensation von:
BOC-Leu-Asp-His-Arg-OH mit H-Gly-Asp-Gly-OEt:
BOC-Leu-Asp-His-Arg-Gly-Asp-Gly-OEt;
BOC-Lys-Gly-Gly-Gly-Asp-Arg-Leu-OH mit H-His-Asp-Gly-OEt:
BOC-Lys-Gly-Gly-Gly-Asp-Arg-Leu-His-Asp-Gly-OEt;
BOC-Lys-Pro-Ser-Asp-OH mit H-Gly-Arg-Gly-OEt:
BOC-Lys-Pro-Ser-Asp-Gly-Arg-Gly-OEt;
BOC-Arg-His-OH mit H-Asp-Leu-OMe:
BOC-Arg-His-Asp-Leu-OMe;
BOC-D-Lys-D-Pro-D-Ser-D-Asp-D-Gly-OH mit H-D-Arg-D-Gly-OMe:
BOC-D-Lys-D-Pro-D-Ser-D-Asp-D-Gly-D-Arg-D-Gly-OMe;
BOC-Leu-Asp-His-OH mit H-Arg-Gly-Asp-OMe:
BOC-Leu-Asp-His-Arg-Gly-Asp-OMe;
BOC-Leu-Asp-His-OH mit H-Arg-Gly-OMe:
BOC-Leu-Asp-His-Arg-Gly-OMe;
BOC-Gly-Arg-His-OH mit H-Asp-Leu-Leu-OMe:
BOC-Gly-Arg-His-Asp-Leu-Leu-OMe;
BOC-Arg-Gly-OH mit H-Asp-Leu-OMe:
BOC-Arg-Gly-Asp-Leu-OMe;
BOC-Cys-Gly-Gly-Gly-Asp-Arg-OH mit H-Leu-His-Gly-OMe:
BOC-Cys-Gly-Gly-Gly-Asp-Arg-Leu-His-Gly-OMe;
BOC-Cys-Gly-Gly-Gly-Asp-Gly-Arg-OH mit H-His-Asp-Ile-OMe:
BOC-Cys-Gly-Gly-Gly-Asp-Gly-Arg-His-Asp-Ile-OMe.

### Beispiel 4

1,3 g BOC-Asp-Gly-Arg-His-Asp-Leu-OMe werden in 60 ml Methanol gelöst, mit 1,5 ml 2 N NaOH-Lösing versetzt und 4 Stunden bei 25° gerührt. Nach Entfernung des Lösungsmittels wird der Rückstand in Wasser aufgenommen, der pH-Wert durch Zugabe von verdünnter HCl auf 3 eingestellt und mit Ethylacetat extrahiert. Der Extrakt wird über Na₂SO₄ getrocknet. Nach Entfernung des Lösungsmittels erhält man BOC-Asp-Gly-Arg-His-Asp-Leu-OH, welches man in 20 ml 2 N HCl auf Dioxanbasis aufnimmt und 2 Stunden bei Raumtemperatur rührt. Die Reaktionsmischung wird zur Trockne eingeengt und der Rückstand durch HPLC gereinigt. Man erhält H-Asp-Gly-Arg-His-Asp-Leu-OH; RZ = 9,5; M⁺ 712.

Analog erhält man durch Entfernung der Schutzgruppen ausgehend von den Verbindungen aus Beispiel 1:
H-Gly-Arg-His-Asp-Leu-OH; RZ = 9,2; M⁺ 597;
H-Asn-Gly-Asp-Gly-Arg-His-Asp-Leu-OH; RZ = 11,3; M⁺ 883;
H-Val-Asn-Gly-Asp-Gly-Arg-His-Asp-Leu-OH; RZ = 12,9; M⁺ 982;
H-Asp-Val-Asn-Gly-Asp-Gly-Arg-His-Asp-Leu-OH; RZ = 13,4; M⁺ 1097;
H-Lys-Gly-Gly-Gly-Asp-Gly-Arg-His-Asp-Leu-OH; RZ = 11,5; M⁺ 1011;
H-Tyr-Gly-Gly-Gly-Asp-Gly-Arg-His-Asp-Leu-OH; RZ = 13,8; M⁺ 1046;
H-Ala-Asp-Gly-Arg-His-Asp-Leu-OH; RZ = 10,7; M⁺ 783;
H-D-Ala-Asp-Gly-Arg-His-Asp-Leu-OH; RZ = 11,1; M⁺ 783;
H-Gly-Asp-Ala-Arg-His-Asp-Leu-OH; RZ = 11,0; M⁺ 784;
H-Gly-D-Asp-Gly-Arg-His-Asp-Leu-OH; RZ = 10,5; M⁺ 770;
H-Gly-Asp-D-Ala-Arg-His-Asp-Leu-OH; RZ = 11,9; M⁺ 784;
H-Gly-Asp-Arg-His-Asp-Leu-OH; RZ = 8,1; M⁺ 712;
H-Cys(Trt)-Gly-Gly-Gly-Asp-Arg-His-Asp-Leu-OH; RZ = 27,7; M⁺ 1171;
H-Cys-Gly-Gly-Gly-Asp-Arg-His-Asp-Leu-OH; RZ = 11,7; M⁺ 929;
H-Cys(Trt)-Gly-Gly-Gly-Asp-Gly-Arg-His-Asp-Leu-OH; RZ = 27; M⁺ 1228;
H-Cys-Gly-Gly-Gly-Asp-Gly-Arg-His-Asp-Leu-OH; RZ = 11,7; M⁺ 987;
H-Cys-Gly-Gly-Thr-Asp-Val-Asn-Gly-Asp-Gly-Arg-His-Asp-Leu-OH; RZ = 12,7; M⁺ 1415;
H-Thr-Asp-Val-Asn-Gly-Asp-Gly-Arg-His-Asp-Leu-OH.

### Beispiel 5

Analog Beispiel 4 erhält man durch Verseifung und Abspaltung der BOC-Schutzgruppen ausgehend von den Verbindungen aus Beispiel 2:
H-Gly-Asp-Gly-Arg-His-Asp-OH; RZ = 3,5; M⁺ 656;
H-Gly-Asp-Gly-Arg-His-OH; RZ = 3,5; M⁺ 541;
H-Gly-Asp-Gly-Arg-His-Asn-Leu-OH; RZ = 11,2; M⁺ 769;
H-Gly-Asp-Gly-Arg-Gly-Asp-Leu-OH; RZ = 11,4; M⁺ 689;
H-Gly-Asp-Gly-Arg-His-D-Asp-Leu-OH; RZ = 11,7; M⁺ 769;
H-Gly-Asp-Gly-Arg-D-His-Asp-Leu-OH; RZ = 11,3; M⁺ 769;
H-Gly-Asp-Gly-D-Arg-His-Asp-Leu-OH; RZ = 10,9; M⁺ 769.
H-Gly-Asn-Gly-Arg-His-Asn-Leu-OH; RZ = 10,8; M⁺ 767;
H-Gly-Asn-Gly-Arg-His-Asp-Leu-OH; RZ = 11,6; M⁺ 768.

### Beispiel 6

Analog Beispiel 4 erhält man ausgehend von den Verbindungen aus Beispiel 3 durch Verseifung und Abspaltung der BOC-Schutzgruppen:
H-Leu-Asp-His-Arg-Gly-Asp-Gly-OH; RZ = 11,1; M⁺ 769;
H-Lys-Gly-Gly-Gly-Asp-Arg-Leu-His-Asp-Gly-OH; RZ = 9,8; M⁺ 1011;
H-Lys-Pro-Ser-Asp-Gly-Arg-Gly-OH; RZ = 3,5; M⁺ 716;
H-Arg-His-Asp-Leu-OH; RZ = 8,0; M⁺ 540;
H-D-Lys-D-Pro-D-Ser-D-Asp-D-Gly-D-Arg-D-Gly-OH; RZ = 24,7; M⁺ 938;
H-Leu-Asp-His-Arg-Gly-Asp-OH; RZ = 11,5; M⁺ 712;
H-Leu-Asp-His-Arg-Gly-OH; RZ = 3,9; M⁺ 597;
H-Gly-Arg-His-Asp-Leu-Leu-OH; RZ = 15,3; M⁺ 710;
H-Arg-Gly-Asp-Leu-OH; RZ = 7,0; M⁺ 460;
H-Cys-Gly-Gly-Gly-Asp-Arg-Leu-His-Gly-OH; RZ = 7,2; M⁺ 871;
H-Cys-Gly-Gly-Gly-Asp-Gly-Arg-His-Asp-Ile-OH; RZ = 15,1; M⁺ 986.

### Beispiel 7

0,9 g H-Arg-His-Asp-Leu-OH werden in 200 ml wäßrigem DMF gelöst und unter Rühren mit 0,5 g Acetylchlorid, gelöst in 10 ml Dichlormethan, tropfenweise versetzt. Die Reaktionsmischung wird 15 Minuten gerührt und stark eingeengt. Das ausfallende Produkt wird abgetrennt. Man erhält H₃C-CO-Arg-His-Asp-Leu-OH; RZ = 12,4; M⁺ 582.

Analog erhält man durch Acetylierung
von H-Gly-Asp-Gly-Arg-His-OH:
H₃C-CO-Gly-Asp-Gly-Arg-His-OH;
von H-Arg-Gly-Asp-Leu-OH:
H₃C-CO-Arg-Gly-Asp-Leu-OH; RZ = 13,0; M⁺ 502;
von H-Lys-Pro-Ser-Asp-Gly-Arg-Gly-OH:
H₃C-CO-Lys-Pro-Ser-Asp-Gly-Arg-Gly-OH
von H-Arg-His-Asp-Leu-OH:
H₃C-CO-Arg-His-Asp-Leu-OH;
von H-D-Lys-D-Pro-D-Ser-D-Asp-D-Gly-D-Arg-D-Gly-OH:
H₃C-CO-D-Lys-D-Pro-D-Ser-D-Asp-D-Gly-D-Arg-D-Gly-OH;
von H-Leu-Asp-His-Arg-Gly-Asp-OH:
H₃C-CO-Leu-Asp-His-Arg-Gly-Asp-OH;
von H-Leu-Asp-His-Arg-Gly-OH:
H₃C-CO-Leu-Asp-His-Arg-Gly-OH.

### Beispiel 8

Analog Beispiel 1 erhält man durch Kondensation von H₃C-CO-Gly-Asp-Gly-Arg-His-OH mit H-Asp-Leu-OMe und anschließender Verseifung H₃C-CO-Gly-Asp-Gly-Arg-His-Asp-Leu-OH; RZ = 14,1; M⁺ 811.

### Beispiel 9

2,0 g BOC-Gly-Arg-His-Asp-Leu-OMe werden in 25 ml 4 N Salzsäure auf Dioxanbasis zwei Std. gerührt. Danach engt man die Reaktionsmischung ein, löst den Rückstand in 100 ml DMF und kühlt auf 0° ab. Anschließend werden nacheinander 1 Äquivalent Fmoc-Gly-Gly-Gly-Asp-OH, 1,3 g TBTU und 1,0 ml Triethylamin zugegeben. Die Lösung wird 2 Std. bei 0° und 12 Std. bei Raumtemperatur gerührt. Nach erneutem Einengen gießt man das Konzentrat in eine NaHCO₃-Lösung ein. Das dabei ausfallende Produkt wird abfiltriert, in 50 ml Methanol gelöst, mit 1,5 ml 2 N NaOH-Lösung versetzt, 4 Stunden bei 25° gerührt und wie üblich aufgearbeitet. Man erhält Fmoc-Gly-Gly-Gly-Asp-Gly-Arg-His-Asp-Leu-OH;
RZ = 28,0; M⁺ 1105.

Analog erhält man durch Kondensation von H-Arg-His-Asp-Leu-OMe
mit Fmoc-Gly-OH:
Fmoc-Gly-Arg-His-Asp-Leu-OH;
mit Fmoc-Asn-Gly-Asp-Gly-OH:
Fmoc-Asn-Gly-Asp-Gly-Arg-His-Asp-Leu-OH;
mit Fmoc-Val-Asn-Gly-Asp-Gly-OH:
Fmoc-Val-Asn-Gly-Asp-Gly-Arg-His-Asp-Leu-OH;
mit Fmoc-Asp-Val-Asn-Gly-Asp-Gly-OH:
Fmoc-Asp-Val-Asn-Gly-Asp-Gly-Arg-His-Asp-Leu-OH;
mit Fmoc-Lys-Gly-Gly-Gly-Asp-Gly-OH:
Fmoc-Lys-Gly-Gly-Gly-Asp-Gly-Arg-His-Asp-Leu-OH;
mit Fmoc-Tyr-Gly-Gly-Gly-Asp-Gly-OH:
Fmoc-Tyr-Gly-Gly-Gly-Asp-Gly-Arg-His-Asp-Leu-OH;
mit Fmoc-Ala-Asp-Gly-OH:
Fmoc-Ala-Asp-Gly-Arg-His-Asp-Leu-OH;
mit Fmoc-D-Ala-Asp-Gly-OH:
Fmoc-D-Ala-Asp-Gly-Arg-His-Asp-Leu-OH;
mit Fmoc-Gly-Asp-Ala-OH:
Fmoc-Gly-Asp-Ala-Arg-His-Asp-Leu-OH;
mit Fmoc-Gly-D-Asp-Gly-OH:
Fmoc-Gly-D-Asp-Gly-Arg-His-Asp-Leu-OH;
mit Fmoc-Gly-Asp-D-Ala-OH:
Fmoc-Gly-Asp-D-Ala-Arg-His-Asp-Leu-OH;
mit Fmoc-Gly-Asp-OH:
Fmoc-Gly-Asp-Arg-His-Asp-Leu-OH;
mit Fmoc-Cys(Trt)-Gly-Gly-Gly-Asp-OH:
Fmoc-Cys(Trt)-Gly-Gly-Gly-Asp-Arg-His-Asp-Leu-OH;
mit Fmoc-Cys-Gly-Gly-Gly-Asp-OH:
Fmoc-Cys-Gly-Gly-Gly-Asp-Arg-His-Asp-Leu-OH;
mit Fmoc-Cys(Trt)-Gly-Gly-Gly-Asp-Gly-OH:
Fmoc-Cys(Trt)-Gly-Gly-Gly-Asp-Gly-Arg-His-Asp-Leu-OH;
mit Fmoc-Cys-Gly-Gly-Gly-Asp-Gly-OH:
Fmoc-Cys-Gly-Gly-Gly-Asp-Gly-Arg-His-Asp-Leu-OH;
mit Fmoc-Cys-Gly-Gly-Thr-Asp-Val-Asn-Gly-Asp-Gly-OH: 
Fmoc-Cys-Gly-Gly-Thr-Asp-Val-Asn-Gly-Asp-Gly-Arg-His-Asp-
Leu-OH;
mit Fmoc-Thr-Asp-Val-Asn-Gly-Asp-Gly-OH:
Fmoc-Thr-Asp-Val-Asn-Gly-Asp-Gly-Arg-His-Asp-Leu-OH.

### Beispiel 10

0,7 g BOC-Gly-Asp-Gly-Arg(BOC)-His-Asp-Leu-OH werden in 100 ml Dichlormethan gelöst, mit 1,4 Äquivalenten MBHA-Harz versetzt und 24 Stunden bei Raumtemperatur gerührt. Nach Entfernung des Lösungsmittels erhält man BOC-Gly-Asp-Gly-Arg(BOC)-His-Asp-Leu-MBHA-Harz, welches man in 20 ml 2 N HCl auf Dioxanbasis aufnimmt und 2 Stunden bei Raumtemperatur rührt. Anschließende Behandlung mit TFA liefert H-Gly-Asp-Gly-Arg-His-Asp-Leu-NH₂; RZ = 8,8; M⁺ 768.

Analog erhält man durch Umsetzung mit MBHA-Harz die folgenden Peptidamide:
aus BOC-Arg(BOC)-His-Asp-Leu-OH:
H-Arg-His-Asp-Leu-NH₂; RZ = 5,4; M⁺ 539;
aus BOC-Arg(BOC)-Gly-Asp-Leu-OH:
H-Arg-Gly-Asp-Leu-NH₂; RZ = 4,7; M⁺ 459;
aus BOC-Gly-Asp-Gly-Arg(BOC)-Gly-Asp-Leu-OH:
H-Gly-Asp-Gly-Arg-Gly-Asp-Leu-NH₂.

### Beispiel 11

Analog Beispiel 7 erhält man ausgehend von H-Gly-Asp-Gly-Arg-His-Asp-Leu-NH₂ durch Acetylierung des Peptids H₃C-CO-Gly-Asp-Gly-Arg-His-Asp-Leu-NH₂; RZ = 12,4; M⁺ 810.

Analog erhält man:
aus H-Arg-His-Asp-Leu-NH₂:
H₃C-CO-Arg-His-Asp-Leu-NH₂; RZ = 11,1; M⁺ 581;
aus H-Arg-Gly-Asp-Leu-NH₂:
H₃C-CO-Arg-Gly-Asp-Leu-NH₂; RZ = 11,3; M⁺ 501.

### Beispiel 12

80 mg H-Thr-Asp-Val-Asn-Gly-Asp-Gly-Arg-His-Asp-Leu-OH werden drei- bis viermal in 0,01 m HCl gelöst und nach jedem Lösevorgang gefriergetrocknet. Anschließende Reinigung durch HPLC liefert H-Thr-Asp-Val-Asn-Gly-Asp-Gly-Arg-His-Asp-Leu-OH × HCl.

Analog erhält man
aus H-Thr-Asp-Val-Asn-Gly-Asp-Gly-Arg-His-Asp-Leu-OH durch Behandlung mit TFA:
H-Thr-Asp-Val-Asn-Gly-Asp-Gly-Arg-His-Asp-Leu-OH × TFA; RZ = 11,8; M+ 1198.

### Beispiel 13

Zur Herstellung von Affinitätsphasen suspendiert man 0,9g N-Maleinimido-C₅H₁₀-CO-NH-C₃H₆-Polymer [erhältlich durch Kondensation von N-Maleinimido-C₅H₁₀-COOH mit H₂N-C₃H₆-Polymer] in 10 ml 0,1 M Natriumphosphatpuffer bei pH 7 und fügt bei 4° 1 Äquivalent H-Cys-Gly-Gly-Gly-Asp-Arg-His-Asp-Leu-OH hinzu. Man rührt 4 Stunden bei gleichzeitiger Erwärmung der Reaktionsmischung auf Raumtemperatur, filtriert den festen Rückstand ab und wäscht zweimal mit je 10 ml Pufferlösung (pH 7) und anschließend dreimal mit je 10 ml Wasser. Man erhält H-Cys[3-(N-Maleinimido-C₅H₁₀-CO-NH-C₃H₆-Polymer)]-Gly-Gly-Gly-Asp-Arg-His-Asp-Leu-OH.

### Beispiel 14

Analog Beispiel 1 erhält man durch Kondensation von Polymer-O-C₃H₆-NH₂ [im Handel erhältlich] und HOOC-C₄H₈-CO-Gly-Asp-Gly-Arg-His-Asp-Leu-OMe [erhältlich durch Kondensation von Adipinsäure mit H-Gly-Asp-Gly-Arg-His-Asp-Leu-OMe unter den genannten Bedingungen] die folgende polymere Phase: Polymer-O-C₃H₆-NH-CO-C₄H₈-CO-Gly-Asp-Gly-Arg-His-Asp-Leu-OMe. Hieraus erhält man durch Verseifung in Methanol mit 2 N NaOH-Lösung gemäß Bsp. 4 Polymer-O-C₃H₆-NH-CO-C₄H₆-CO-Gly-Ap-Gly-Arg-His-Asp-Leu-OH.

Die nachstehenden Beispiele betreffen pharmazeutische Zubereitungen.

### Beispiel A: Injektionsgläser

Eine Lösung von 100 g eines Wirkstoffes der Formel I und 5 g Dinatriumhydrogenphosphat in 3 l zweifach destilliertes Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojolecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9,38 g NaH₂PO₄ × 2 H₂O, 28,48 g Na₂HPO₄ × 12 H₂O und 0,1 g Benzalkoniumchlorid in 940 ml zweifach destilliertem Wasser. Man stellt auf pH 6,8 ein, füllt auf 1 l auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Lineare Peptide der Formel I
X-A-B-C-Arg-E-G-L-Z I,
worin
X H, Acyl mit 1-10 C-Atomen, H-Asn, H-Val-Asn, H-Asp-Val-Asn, Fmoc-Gly-Gly, H-Lys-Gly-Gly, H-Lys-Pro, H-Tyr-Gly-Gly, H-Cys-Gly-Gly, H-Cys(Trt)-Gly-Gly, H-Cys-Gly-Gly-Thr-Asp-Val-Asn oder H-Thr-Asp-Val-Asn,
A, B und C jeweils unabhängig voneinander fehlen oder jeweils einen Aminosäurerest, ausgewählt aus einer Gruppe bestehend aus Ala, Arg, Asn, Asp, Asp(OR), Cys, Gln, Glu, Gly, His, Ile, Leu, Lys, Lys(Ac), Lys(AcNH₂), Lys(AcSH), Met,Orn, Phe, 4-Hal-Phe, Pro, Ser, Thr, Trp, Tyr oder Val, wobei die genannten Aminosäurereste auch derivatisiert sein können,
E Gly, His oder Leu-His,
G fehlt oder Asp oder Asn,
L fehlt oder Gly, Ile, Leu oder Leu-Leu,
Z NH₂ oder OH,
Hal F, Cl, Br, I und
Ac Alkanoyl mit 1-10 C-Atomen
bedeuten,
sowie deren physiologisch unbedenklichen Salze.

2. Ein Enantiomer oder ein Diasteromer einer Verbindung der Formel I gemäß Anspruch 1.

3. (a) H-Gly-Asp-Gly-Arg-His-Asp-Leu-OH;
(b) H-Thr-Asp-Va-Asn-Gly-Asp-Gly-Arg-His-Asp-Leu-OH;
(c) H-Lys-Gly-Gly-Gly-Asp-Gly-Arg-His-Asp-Leu-OH;
(d) Fmoc-Gly-Gly-Gly-Asp-Gly-Arg-His-Asp-Leu-OH;
(e) CH₃-CO-Gly-Asp-Gly-Arg-His-Asp-Leu-OH;
(f) H-Cys-Gly-Gly-Asp-Arg-His-Asp-Leu-OH;
(g) H-Gly-Arg-His-Asp-Leu-OH.

4. Verfahren zur Herstellung einer Verbindung der Formel I nach Anspruch 1 oder eines ihrer Salze, dadurch gekennzeichnet, daß man sie aus einem ihrer funktionellen Derivate durch Behandeln mit einem solvolysierenden oder hydrogenolysierenden Mittel in Freiheit setzt
oder daß man eine Verbindung der Formel II
M-Z II,
worin
Z die angegebene Bedeutung besitzt und
M
(a) X, aber nicht Wasserstoff, Fmoc-Gly, H-Lys-Gly, H-Lys, H-Tyr-Gly, H-Asp-Val, H-Tyr, H-Val, H-Asp, H-Cys-Gly-Gly-Thr-Asp-Val, H-Cys-Gly-Gly-Thr-Asp, H-Cys-Gly-Gly-Thr, H-Cys-Gly-Gly, H-Cys-Gly, H-Cys, H-Cys(Trt)-Gly, H-Cys(Trt), H-Thr-Asp-Val, H-Thr-Asp oder H-Thr,
(b) X-A,
(c) X-A-B,
(d) X-A-B-C,
(e) X-A-B-C-Arg,
(f) X-A-B-C-Arg-E oder
(g) X-A-B-C-Arg-E-G
bedeutet,
mit einer Aminoverbindung der Formel III
H-Q-Z III
worin
Z die angegebene Bedeutung besitzt und
Q
(a) H-A-B-C-Arg-E-G-L, H-Asn-A-B-C-Arg-E-G-L, H-Val-Asn-A-B-C-Arg-E-G-L, H-Pro-A-B-C-Arg-E-G-L, H-Gly-A-B-C-Arg-E-G-L, H-Gly-Gly-A-B-C-Arg-E-G-L, H-Asn-A-B-C-Arg-E-G-L, H-Val-Asn-A-B-C-Arg-E-G-L, H-Asp-Val-Asn-A-B-C-Arg-E-G-L, H-Thr-Asp-Val-Asn-A-B-C-Arg-E-G-L, H-Gly-Thr-Asp-Val-Asn-A-B-C-Arg-E-G-L oder H-Gly-Gly-Thr-Asp-Val-Asn-A-B-C-Arg-E-G-L,
(b) H-B-C-Arg-E-G-L,
(c) H-C-Arg-E-G-L,
(d) H-Arg-E-G-L,
(e) H-E-G-L,
(f) H-G-L oder
(g) H-L
bedeutet,
umsetzt,
und/oder daß man gegebenenfalls eine freie Aminogruppe acyliert und/oder eine Verbindung der Formel I durch Behandeln mit einer Säure oder Base in eines ihrer Salze überführt.

5. Verfahren zur Herstellung pharmazeutischer Zubereitungen, dadurch gekennzeichnet, daß man eine Verbindung der Formel I nach Anspruch 1 und/oder eines ihrer physiologisch unbedenklichen Salze zusammen mit mindestens einem festen, flüssigen oder halbflüssigen Träger- oder Hilfsstoff in eine geeignete Dosierungsform bringt.

6. Pharmazeutische Zubereitung, gekennzeichnet durch einen Gehalt an mindestens einer Verbindung der allgemeinen Formel I nach Anspruch 1 und/oder einem ihrer physiologisch unbedenklichen Salze.

7. Verwendung von Verbindungen der Formel I nach Anspruch 1 oder von deren physiologisch unbedenklichen Salzen zur Herstellung eines Arzneimittels zur Bekämpfung von Krankheiten.

8. Verwendung von Verbindungen der Formel I nach Anspruch 1 oder von deren physiologisch unbedenklichen Salzen bei der Bekämpfung von Krankheiten.

9. Verwendung von Verbindungen der Formel I nach Anspruch 1 zur Herstellung von immobilisierten Liganden für die Affinitätssäulenchromatographie.

10. Verwendung von Verbindungen der Formel I nach Anspruch 1 zur Reinigung von Integrinen durch Affinitätschromatographie.
